# EUROPEAN PATENT APPLICATION

(11) **EP 1 847 213 A1**
(43) Date of publication of application: **24.10.2007**
(21) Application number: 06713526.9
(22) Date of filing: 10.02.2006
(51) Int. Cl.: A61B 1/00, C09J 163/02

(54) **ENDOSCOPE DEVICE**

(30) Priority: 10.02.2005 JP 2005034749
(71) Applicant: Olympus Corporation, Tokyo 151-0072 (JP)
(72) Inventor: KAGAWA, Ichiro, Olympus Intellectual Property Services Co., Ltd., Hachioji-shi, Tokyo 192-8512 (JP); MATSUMOTO, Jun, Olympus Intellectual Property Services Co., Ltd., Hachioji-shi, Tokyo (JP)
(74) Representative: von Hellfeld, Axel
(86) International application number: PCT/JP2006/302384
(87) International publication number: WO 2006/085623

(57) **Abstract**

This invention uses a two-part reaction type adhesive obtained by mixing a base compound containing at least one of a bisphenol A type epoxy resin and bisphenol F type epoxy resin and 5 to 15 wt% of a fine acrylic rubber powder having an average particle size of 300 nm or less, and a curing agent containing dimer acid, diethylenetriamine, and a diethylenetriamine monomer as main components.

## Description

### Technical Field

The present invention relates to an endoscope apparatus and, more particularly, to bonding of parts constituting an endoscope by an adhesive.

### Background Art

The diameter of an insertion portion of an endoscope must be decreased as small as possible because the endoscope is inserted into the body cavity or the like, but various contents are inserted into this insertion portion in order to diversify the functions of the endoscope.

Examples of the contents inserted into the insertion portion of the endoscope are a clamp tube for inserting a large treatment tool in order to improve the endoscope treatment using the endoscope, a tube for supplying air into the body cavity in order to reduce the suffering on the patient, and a tube for washing the surface of a lens incorporated into a distal-end hard portion of the insertion portion and washing away garbage in the body cavity, and these tubes are normally made of Teflon, olefin, or silicone. An adhesive is used to fix the entrance portions of these tubes to the distal end or an operation unit.

Also, the distal-end hard portion of the insertion portion contains a cover lens and its lens groups and a cover lens and its lens groups for illumination from a light guide as an optical system for observing the body cavity. An adhesive is used to fix these lens groups to a lens frame or the distal-end hard portion.

In addition, the light guide for transmitting light to the distal end portion and an image guide for transmitting an image to an eyepiece portion are inserted into the insertion portion. These light guide and image guide are fiber bundles formed by binding a large number of fibers. An adhesive is used to fix these fiber bundles to the lens frame or distal-end hard portion.

Furthermore, in an electronic endoscope, a cable or the like that transmits an electrical signal from, e.g., a CCD incorporated into the distal-end hard portion is inserted in addition to the tubes and fiber bundles described above. An adhesive is used to protect and fix the CCD and the like.

In addition to bonding the components of the endoscope described above, after the end portion of a flexible outer tube is fixed to an internal member by externally tying the end portion by a thread, the outer surface of the thread is finished and the thread is fixed by covering it with an adhesive in order to ensure insertion properties and prevent a fray of the thread.

As described above, the diameter of the insertion portion of the endoscope is decreased as small as possible and the various contents are inserted into the insertion portion in order to diversify the endoscope functions, so an adhesive is used to bond these parts because screws and machine screws cannot be used. An epoxy-based adhesive is often used as this adhesive as disclosed in, e.g., Jpn. Pat. Appln. KOKAI Publication No. 2002-238834.

A medical endoscope is inserted into the body cavity of a patient and hence must be completely sterilized, and examples of the sterilizer are an aldehyde-based sterilizer such as glutaraldehyde, hydrogen peroxide, peracetic acid, strongly acidic water alcohol, benzalkonium chloride (an ammonia-based sterilizer), chlorhexidine glucagon (a biguanato-based sterilizer), alkyldiaminoethylglycine hydrochloride (an amphoteric surfactant-based sterilizer), a phenol-based sterilizer, an iodine-based sterilizer, a sodium hypochlorite-based (chlorine-based) sterilizer, and a sodium hydroxide-based sterilizer.

After garbage is washed away with water, the endoscope is sterilized by dipping it into a liquid chemical of the disinfectant described above, or by wiping its surface with gauze impregnated with a liquid sterilizer. Recently, demands have arisen for improving the sterilization level in order to reliably prevent infection between patients via the endoscope. For this purpose, a hydrogen peroxide plasma gas is beginning to be used in addition to an aqueous peracetic acid solution and hydrogen peroxide solution having high sterilizing effects as sterilizers. In addition, autoclave sterilization using saturated steam at 135°C x 23 atm is also beginning to be used. Of these sterilization methods, sterilization using a hydrogen peroxide solution or hydrogen peroxide plasma gas and autoclave sterilization are beginning to be widely used because no harmful residues remain after the sterilization and no ventilation facilities are required unlike when formalin gas or ethylene oxide gas is used.

As the sterilization and disinfection methods diversify and the number of types of liquid chemicals increases accordingly, however, the conventional epoxy adhesives used to bond the endoscope parts have become unsuitable for some sterilization and disinfection methods because brittle fracture occurs or the adhesive strength decreases due to, e.g., boiling water and saturated steam of boiling disinfection and autoclave sterilization, active oxygen of peracetic acid, the acidic substance of strongly acidic water, and the hydrogen peroxide plasma gas. This makes it difficult to maintain the performance of the endoscope if it is used for a long time.

Since oxidation deterioration, heat softening, cure aging, and hydrolysis of the adhesive are presumably the causes of these problems, it is necessary to develop an adhesive having improved the oxidation deterioration resistance, heat aging resistance, and hydrolysis resistance while maintaining the present chemical resistance.

Accordingly, demands have arisen for an endoscope using an adhesive improved in oxidation deterioration resistance, heat aging resistance, and hydrolysis resistance, and having resistances to all sterilization and disinfection methods.

Since the temperature of hot steam of autoclave sterilization is about 135°C, it is necessary to use endoscope parts having a heat resistance equivalent to this temperature. Also, an adhesive for bonding the endoscope parts must have an equivalent heat resistance. An epoxy adhesive changes into a cured product when a base compound made of an epoxy resin is cured with a curing agent made of an acid anhydride-based or amine-based material.

That is, when assembling an endoscope, its parts are preferably bonded by curing an adhesive at a temperature as low as possible for a time as short as possible. Also, since the materials of the parts are metals, plastics, rubber, and the like, the adhesive favorably has constant adhesive force from hard materials to soft materials. From the foregoing, an adhesive that cures at 60°C to 80°C and has a heat resistance of 135°C is desirable.

To increase the chemical resistance and heat resistance of an adhesive, it is necessary to make the adhesive very hard by increasing the crosslink density or making the glass transition point of the cured epoxy resin 100°C or higher.

As described above, however, an adhesive must have high adhesive force from hard parts to soft parts, and no high adhesive strength is obtained, especially when bonding soft parts, by increasing the crosslink density or by using a hard cured epoxy resin having a glass transition point of 100°C or higher.

That is, an adhesive for use in an endoscope apparatus must be hard and soft at the same time, i.e., must have incomparable properties, since it is necessary to increase not only the shear strength but also the peel strength.

### Disclosure of Invention

It is an object of the present invention to provide an endoscope apparatus capable of maintaining the performance for long time periods by adhering and fixing parts by using a highly durable adhesive that does not decrease the adhesive strength by various disinfection methods, particularly, treatments in environments such as saturated steam, active oxygen of peracetic acid, the acidic substance of strongly acidic water, and a hydrogen peroxide plasma gas.

First, the present invention provides an endoscope apparatus in which parts constituting an endoscope are bonded by an adhesive, characterized in that the adhesive is a two-part reaction type adhesive obtained by mixing a base compound in which a fine acrylic rubber powder having an average particle size of 300 nm or less is dispersed in at least one bisphenol-based epoxy resin selected from the group consisting of a bisphenol A type epoxy resin and a bisphenol F type epoxy resin such that an amount of the fine acrylic rubber powder is 5 to 15 wt% with respect to a weight of the epoxy resin, and a curing agent containing dimer acid, diethylenetriamine, and a diethylenetriamine monomer as main components.

Second, the present invention provides an endoscope apparatus in which an end portion of a flexible outer tube of an insertion portion of an endoscope is fixed to an internal member by externally tying the end portion by a thread, and an outer surface of the thread is finished and the thread is fixed by coating the thread with an adhesive in order to ensure insertion properties and prevent a fray of the thread, characterized in that the adhesive is a two-part reaction type adhesive obtained by mixing a base compound in which a fine acrylic rubber powder having an average particle size of 300 nm or lass is dispersed in at least one bisphenol-based epoxy resin selected from the group consisting of a bisphenol A type epoxy resin and a bisphenol F type epoxy resin such that an amount of the fine acrylic rubber powder is 5 to 15 wt% with respect to a weight of the epoxy resin, and a curing agent containing dimer acid, diethylenetriamine, and a diethylenetriamine monomer as main components.

Third, the present invention provides an endoscope apparatus in which an adhesive is used to raise an adhesive layer around one of an observation lens and an illumination lens of an endoscope, thereby smoothing a corner portion of an edge of the lens, characterized in that the adhesive is a two-part reaction type adhesive obtained by mixing a base compound in which a fine acrylic rubber powder having an average particle size of 300 nm or less is dispersed in at least one bisphenol-based epoxy resin selected from the group consisting of a bisphenol A type epoxy resin and a bisphenol F type epoxy resin such that an amount of the fine acrylic rubber powder is 5 to 15 wt % with respect to a weight of the epoxy resin, and a curing agent containing dimer acid, diethylenetriamine, and a diethylenetriamine monomer as main components.

### Brief Description of Drawings

FIG. 1 is a view showing an outline of the arrangement of an example of an endoscope apparatus according to the present invention;
FIG. 2 is a view showing an example of an insertion portion applicable to FIG. 1; and
FIG. 3 is a view showing another example of the insertion portion applicable to FIG. 1.

### Best Mode for Carrying Out the Invention

An endoscope apparatus of the present invention is an endoscope apparatus in which parts constituting an endoscope are bonded by a specific adhesive, wherein the adhesive is a two-part reaction type adhesive obtained by mixing a base compound in which a fine acrylic rubber powder having an average particle size of 300 nm or less is dispersed in at least one bisphenol-based epoxy resin selected from the group consisting of a bisphenol A type epoxy resin and a bisphenol F type epoxy resin such that the amount of the fine acrylic rubber powder is 5 to 15 wt% with respect to the weight of the epoxy resin, and a polyamidoamine-based curing agent containing dimer acid, diethylenetriamine, and a diethylenetriamine monomer as main components.

The acrylic rubber is added to the bisphenol-based epoxy resin in the base compound described above in order to modify the bisphenol-based epoxy resin and increase both the adhesive shear strength and adhesive peel strength by blending the acrylic rubber to the bisphenol-based epoxy resin, thereby making it possible to adhere hard metal parts as well as soft rubber parts. For this purpose, the acrylic rubber must be added to the bisphenol-based epoxy resin such that the addition amount is 5 to 15 wt% with respect to the weight of the epoxy resin.

By thus adding the acrylic rubber to the bisphenol-based epoxy resin, it is possible to increase the adhesive shear strength, particularly, the adhesive peel strength of bonded portions adhered by the adhesive.

The addition amount of the fine acrylic rubber powder to the bisphenol-based epoxy resin is 5 to 15 wt%, preferably, 15 wt% with respect to the weight of the epoxy resin. When the concentration of the acrylic rubber falls within the above range, it is possible to obtain an adhesive layer superior in water resistance and strongly acidic chemical resistance. Also, when the fine acrylic rubber powder is dispersed in the bisphenol-based epoxy resin such that the amount of the fine powder is 5 to 15 wt% with respect to the weight of the epoxy resin, it is possible to increase the shear adhesive strength, particularly, the peel adhesive strength. It is experimentally found that the effect of imparting elasticity cannot be obtained if this amount is 5 wt% or less, and the water resistance extremely decreases if the amount exceeds 15 wt%. Furthermore, if the addition amount of the fine acrylic rubber powder falls outside the above range, the autoclave resistance and chemical resistance sometimes decrease.

The acrylic rubber used in the present invention is preferably dispersed as a fine powder having an average particle size of 300 nm or less in an epoxy resin, and the dispersion can be mixed in a base compound, curing agent, and the like after that. When the adhesive containing a mixture of the bisphenol-based epoxy resin and acrylic rubber as a base compound is heated to cause a curing reaction, the adhesive forms a sea island structure in which the acrylic rubber distributes in the form of islands in the bisphenol-based epoxy resin, and reveals various characteristics on the basis of this sea island structure. However, if a rubber material is added in the form of a liquid to this sea island structure, the sea island structure strongly depends upon the mixing/curing conditions and the like, and the desired adhesive characteristics can be obtained under only extremely limited conditions. By contrast, when the acrylic rubber is predispersed in the form of a fine powder in the epoxy resin, the sea island structure hardly depends upon the mixing/curing conditions and is readily controllable. This makes it possible to increase the degree of freedom of, e.g., the adhering work and curing conditions. A bisphenol A type epoxy resin or bisphenol F type epoxy resin in which 5 to 15 wt% of this fine acrylic rubber powder are dispersed and a mixture of the epoxy resin can adhere hard metal parts as well as soft rubber parts.

The average particle size of the acrylic rubber is preferably 50 to 300 nm, and fine stress deformation can be absorbed if the average particle size falls within this range. The average particle size is more preferably 150 to 300 nm. If the average particle size is less than 50 nm, it becomes often impossible to absorb fine stress cracks. If the average particle size exceeds 300 nm, the sizes of the islands of the sea island structure increase, and the resin often softens. Note that the average particle size herein mentioned is a volume-average particle size.

The curing agent used in the present invention is a polyamidoamine-based curing agent comprising dimer acid, diethylenetriamine, and a diethylenetriamine monomer.

Many polyamine-based curing agents generally have a low heat resistance and low chemical resistance, particularly, a low strongly acidic chemical resistance. This is so because active oxygen of a strongly acidic liquid chemical attacks amine bonds and deteriorates an adhesive. However, curing agents such as acid anhydride-based and phenol-based curing agents other than the polyamine-based curing agents normally require high curing temperatures of 120°C to 150°C. The polyamine-based curing agents are more advantageous in low-temperature curability than those curing agents, and the adhesive strength of the polyamine-based curing agents is higher than that of those curing agents.

By contrast, the present invention can increase the strongly acidic chemical resistance by using, instead of the polyamine-based curing agent, a polyamidoamine-based curing agent in which diethylenetriamine is applied to an amine portion of the polyamine-based curing agent, thereby increasing all the low-temperature curability, adhesive strength, and strongly acidic chemical resistance of the epoxy adhesive.

Furthermore, it was found by conducting many experiments that high adhesive strength is obtained when the blending amount of the curing agent with respect to the bisphenol-based epoxy resin in the base compound is 80% to 120% of a theoretical blending ratio calculated from an epoxy equivalent weight and amine equivalent weight, i.e., when the blending ratio of the polyamidoamine-based curing agent with respect to 100 parts by weight of the bisphenol-based epoxy resin is 25 to 36 parts by weight. The molecular weight per functional group of an epoxy resin is called an epoxy equivalent weight, the amine equivalent weight of an amine-based curing agent is also called an active hydrogen equivalent weight, and the theoretical blending ratio is calculated from the epoxy equivalent weight and amine equivalent weight.

As described above, optimizing the blending ratio of the bisphenol-based epoxy resin to diethylenetriamine-based curing agent further increases the heat resistance and chemical resistance, particularly, the strongly acidic chemical resistance, and this makes it possible to maintain high adhesive strength even in a saturated steam environment at 135°C x 23 atm, peracetic acid environment, and hydrogen peroxide plasma environment. Note that if the blending ratio of the polyamidoamine-based curing agent to 100 parts by weight of the bisphenol-based epoxy resin is less than 25 parts by weight, no sufficient adhesive strength is obtained, and the adhesive layer sometimes causes oxidation deterioration, softening deterioration and cure aging by heat, hydrolysis, and brittle fracture, and deceases the adhesive strength when exposed to active oxygen in peracetic acid, an acidic substance in strongly acidic water, a hydrogen peroxide plasma gas, boiling water of boiling disinfection, and hot steam of autoclave sterilization. In addition, no desired characteristics can be obtained in some cases because an unreacted epoxy resin becomes excessive. If the blending ratio of the polyamidoamine-based curing agent exceeds 36 parts by weight, unreacted amine becomes excessive, and free amine remains. This decreases the water resistance, and the adhesive layer often causes oxidation deterioration, softening deterioration and cure aging by heat, hydrolysis, and brittle fracture, and deceases the adhesive strength when exposed to active oxygen in peracetic acid, an acidic substance in strongly acidic water, a hydrogen peroxide plasma gas, boiling water of boiling disinfection, and hot steam of autoclave sterilization.

In the present invention, the above-mentioned two-part reaction type adhesive can further contain additives such as a catalyst, adhesion imparting agent, solvent, plasticizer, filler, antioxidant, polymerization inhibitor, surfactant, fungiproof agent, and coloring agent, in addition to the base compound and curing agent described above. An adhesive applicable to the present invention can be blended by using various apparatuses that manufacture ordinary adhesives. These additives can be added to the base compound or to the mixture of the base compound and curing agent. For example, the above two-part reaction type adhesive can further contain, as an adhesive imparting agent or the like, 0.1 to 3 parts by weight of epoxy silane with respect to 100 parts by weight of the base compound. Also, the above three-part reaction type adhesive can further contain, as a filler and the like, 40 to 50 parts by weight of a spherical molten silica powder and 10 to 20 parts by weight of an ultrafine spherical silica powder with respect to 100 parts by weight of the base compound.

The parts of an endoscope, for example, are adhered as follows by using the two-part reaction heat-sensitive adhesive explained above.

That is, a solution A containing the base compound and a solution B containing the curing agent are mixed at a predetermined ratio. Then, the surfaces of predetermined endoscope parts to which this mixture is to be applied are coated with the mixture by using a brush or the like, and the two parts are bonded, fixed, and heated at a predetermined heating temperature for a predetermined heating time, thereby strongly adhering the two endoscope parts.

Similarly, it is also possible to seal an imaging device of the endoscope, finish the outer surface of an end portion of a flexible outer tube and fix the end portion, and pour an adhesive layer around an observation lens or illumination lens.

The heating temperature is preferably 60°C to 135°C, although it depends on the types and blending ratio of the base compound and curing agent of the adhesive. The heating time is preferably about 0.5 to 3 hrs. The curing reaction progresses slowly and takes a long time if the heating temperature is lower than 60°C, and endoscope parts having a low heat resistance often thermally deteriorates if the heating temperature exceeds 135°C .

The present invention will be explained in more detail below with reference to the accompanying drawings.

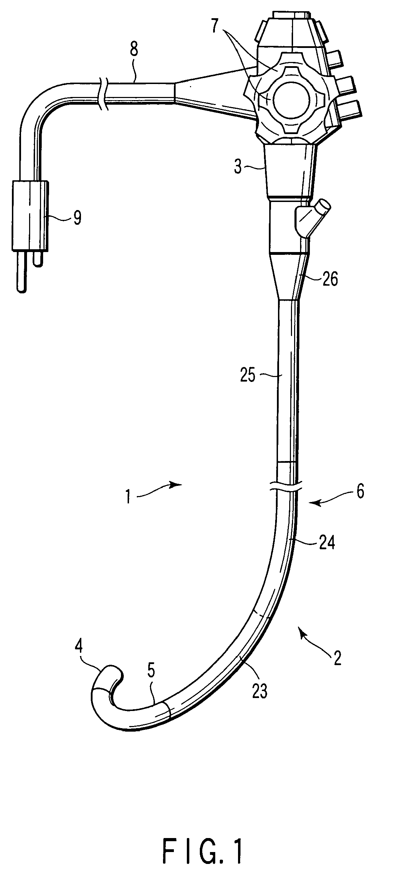

FIG. 1 shows an outline of the overall arrangement of a soft mirror 1 as an example of the endoscope apparatus of the present invention.

In this soft mirror 1, an operation unit 3 on the operator side is connected to the proximal end portion of an elongated insertion portion 2 to be inserted into the body. The insertion portion 2 is formed by sequentially connecting a distal end portion 4, curved portion 5, and elongated flexible tube 6. The curved portion 5 can be curved, e.g., vertically and horizontally.

An operation knob 7 for curving the curved portion 5, e.g., vertically and horizontally is attached to the operation unit 3, and the proximal end portion of a universal cord 8 is connected to the operation unit 3. A connector 9 is attached to the distal end portion of the universal cord 8. The connector 9 is detachably attached to a light source device (not shown).

In the present invention, members to be bonded by using the two-part reaction type adhesive are not particularly limited as long as they are the components of the endoscope apparatus. For example, it is possible by using the above-mentioned two-part reaction type adhesive to fix the entrance portions of various tubes to be inserted into the insertion portion of the endoscope apparatus to the distal end of the insertion portion or to the operation unit, fix lens groups arranged in a distal-end hard portion of the insertion portion to a lens frame or the distal-end hard portion, fix fiber bundles inserted into the insertion portion to the lens frame or distal-end hard portion, and protect, fix, and seal a CCD or the like of an imaging device incorporated into the distal-end hard portion.

Also, after the end portion of a flexible outer tube of the insertion portion of the endoscope apparatus is fixed to an internal member by externally tying the end portion by a thread, the thread may be coated with the above two-part reaction type adhesive. When the thread is thus coated with the adhesive, it is possible to ensure insertion properties by finishing the outer surface of the thread with the adhesive, and prevent a fray of the thread at the same time.

Furthermore, it is possible to seal an imaging element of the endoscope apparatus, or raise the adhesive around an observation lens or illumination lens of the endoscope apparatus to smooth the corner portion of the edge of the lens.

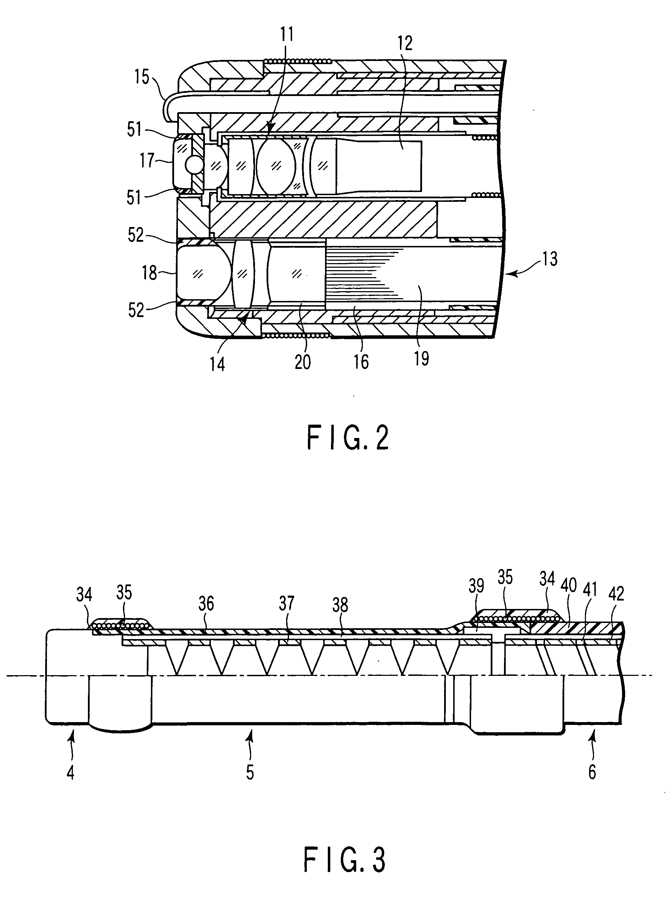

FIG. 2 is a sectional view showing an outline of the arrangement of the distal end portion 4 used in an example of the insertion portion 2 applicable to the soft mirror 1 shown in FIG. 1. As shown in FIG. 1, the distal end portion 4 comprises an objective lens 11 for observing the interior of the body, a CCD unit 12 placed on the image formation plane of the objective lens 11, a light guide 13 for illuminating the observation surface, an illumination lens 14 for spreading the illuminating light from the light guide 13, and an air/water supply nozzle 15 for spraying water when the front surface of the objective lens 11 becomes dirty, and for supplying air.

In the distal end portion 4, the two-part reaction type adhesive described above can be used to, e.g., fix and seal a fiber bundle 19 in the light guide 13 to a lens frame 20, distal-end hard portion 16, and the like, as indicated by adhesives 51 and 52. In addition, the above-mentioned two-part reaction type adhesive can be used to, e.g., fix the air/water supply nozzle 15 to the distal-end hard portion 16 and the like.

FIG. 3 shows another example of the insertion portion 2 applicable to the soft mirror 1 shown in FIG. 1.

In the insertion portion 2, a curved portion 2 is connected to the distal end of a flexible tube 3 via a connection tube 39, and the distal end portion 4 including a camera unit incorporating an optical system (not shown) and the like is connected to the distal end of the curved portion 2. The curved portion 2 can be bent by a remote operation.

The flexible tube 3 is formed by covering, with a metal mesh tube 41, the outer surface of a spiral tube 42 obtained by winding a metal band, and further covering the outer surface of the metal mesh tube 41 with a flexible outer tube 40 made of a resin. The resin forming the flexible outer tube 40 can be any resin as long as it has flexibility and can resist a sterilization treatment.

The curved portion 5 is formed by covering, with a metal mesh tube 38, the outer surfaces of node rings 37 connected to be tiltable, and covering the outer surface of the metal mesh tube 38 with a flexible outer tube 36 made of rubber.

The outer circumferential surfaces of the end portions of both the shell tubes 36 and 40 are tied by threads 35, and fixed to the metal mesh tubes 41 and 38, camera unit 4, or connection tube 39 inside these end portions. Tying by the threads 35 is done by winding the threads 35 around the outer circumferential surfaces of the end portions of the shell tubes 36 and 40. The number of turns is not particularly limited.

The shell tubes 36 and 40 whose outer circumferential surfaces are tied by the threads 35 as described above are coated with the above-mentioned, two-part reaction type adhesive from the outside of the threads 35, thereby forming adhesive layers 34 and preventing a fray of the threads 35. The amount of the adhesive layers 34 is desirably as small as possible so as not to increase the diameter of the insertion portion of the endoscope.

As described above, the present invention bonds parts of an endoscope, finishes the outer surface of a flexible outer tube end portion of an insertion portion of the endoscope and fix a thread, seals an imaging element of the endoscope, or raises an adhesive around an observation lens or illumination lens of the endoscope to smooth a corner portion of the lens edge by using, as the adhesive, a two-part reaction type adhesive obtained by mixing, at a predetermined blending ratio, a base compound formed by adding a predetermined amount of rubber and/or plastic to an epoxy resin, a curing agent made of predetermined amine, and heat type cation-based curing agent. This makes it possible to obtain an endoscope apparatus that maintains high adhesive strength and the initial outer appearance of the end portion of a flexible outer tube regardless of the type of disinfection method.

### Examples

The present invention will be described in detail below by way of its examples.

### Example 1

Sixty parts by weight of bisphenol A type epoxy resin BPA328 (manufactured by NIPPON SHOKUBAI) formed by dispersing 20 parts by weight of acrylic rubber having an average particle size of 300 nm and 10 parts by weight of bisphenol A type epoxy resin were prepared as base compound components, 40 parts by weight of curing agent 2131N (manufactured by ThreeBond) containing dimer acid, diethylenetriamine, and a diethylenetriamine monomer were prepared as curing agent components, the base compound and curing agent were mixed, and 0.14 parts by weight of a silane coupling agent were mixed in the mixture, thereby obtaining a two-part reaction type adhesive for use in the present invention.

Table 1 below shows the component formulation of the obtained adhesive.

Also, the obtained adhesive was used to adhere stainless steel samples (SUS + SUS) and a stainless steel sample and engineering plastic sample by curing the adhesive at 80°C x 2 hrs. The adhered samples were used to measure the initial adhesive strength, and conduct an acidic liquid chemical test (in a peracetic acid environment and hydrogen peroxide solution environment) and an autoclave test. Table 1 below shows the results.

The adhesive strength test was conducted in conformity with JIS K6850 "Method of Testing Tensile Shear Adhesive Strength of Adhesive", and measurements were performed on samples made of stainless steel 304 (SUS + SUS) and samples made of stainless steel 304 and engineering plastic.

The peracetic acid environment test and hydrogen peroxide solution environment test were conducted in conformity with JIS K6858 "Adhesive Chemical Resistance Test". In the peracetic acid environment test, the above samples were dipped in an aqueous 4% peracetic acid solution at 53°C for 24 hrs, and dried at room temperature after being removed from the solution, and the tensile shear adhesive strong test was measured. In the hydrogen peroxide solution environment test, the samples were dipped in an aqueous 30% hydrogen peroxide solution for 100 hrs, and dried at room temperature for 24 hrs after being removed from the solution, and the tensile shear adhesive strong test was measured.

In the autoclave test, 600 samples described above were sterilized in a steam sterilization apparatus using 135°C steam, and dried at room temperature for 24 hrs after being removed from the apparatus, and the tensile shear adhesive strength test was measured. The unit of the adhesive strength is MPa.

Also, endoscope parts were coated with the obtained adhesive and adhered, and the adhesive was cured at 80°C for 2 hrs in this state.

The end portion of a flexible outer tube of an insertion portion as an endoscope part was fixed to an internal member by externally tying the end portion by a thread, and the thread was coated with the adhesive to finish the outer surface of the thread. In addition, the adhesive was used to seal imaging device of the endoscope. Furthermore, the adhesive was used to raise adhesive layers around an observation lens and illumination lens of the endoscope, thereby smoothing corner portions of the edges of the observation lens and illumination lens. In this manner, it was possible to assemble the endoscope apparatus without any problems.

### Example 2

An adhesive was obtained following the same procedures as in Example 1 except that 60 parts by weight of bisphenol F type epoxy resin BPF307 (manufactured by NIPPON SHOKUBAI) formed by dispersing 20 parts by weight of acrylic rubber having an average particle diameter of 300 nm were used as base compound components.

Following the same procedures as in Example 1, the obtained adhesive was used to measure the initial adhesive strength, and conduct an acidic liquid chemical test (in a peracetic acid environment and hydrogen peroxide solution environment) and an autoclave test. Table 1 below shows the results.

It was also possible to assemble an endoscope apparatus in the same manner as in Example 1 by coating endoscope parts with the obtained adhesive.

### Comparative Example 1

One hundred parts by weight of bisphenol A type epoxy resin BPA328 formed by dispersing 20 parts by weight of acrylic rubber having an average particle size of 300 nm were prepared as base compound components, 30 parts by weight of curing agent 2131N (manufactured by ThreeBond) was prepared as curing agent components, 0.14 parts by weight of epoxy silane were prepared as an adhesive imparting agent, and these materials were mixed to obtain an adhesive.

Following the same procedures as in Example 1, the obtained adhesive was used to measure the initial adhesive strength, and conduct an acidic liquid chemical test (in a peracetic acid environment and hydrogen peroxide solution environment) and an autoclave test. Table 1 below shows the results.

It was also possible to assemble an endoscope apparatus in the same manner as in the first embodiment by coating endoscope parts with the obtained adhesive.

### Comparative Example 2

Sixty parts by weight of bisphenol F type epoxy resin BPA328 formed by dispersing 20 parts by weight of acrylic rubber having an average particle size of 300 nm and 10 parts by weight of bisphenol A type epoxy resin were prepared as base compound components, 40 parts by weight of polyamidoamine-based curing agent 290F (manufactured by FUJI KASEI KOGYO) were prepared as curing agent components, 0.14 parts by weight of epoxy silane were prepared as an adhesive imparting agent, and these materials were mixed to obtain an adhesive.

Following the same procedures as in Example 1, the obtained adhesive was used to measure the initial adhesive strength, and conduct an acidic liquid chemical test (in a peracetic acid environment and hydrogen peroxide solution environment) and an autoclave test. Table 1 below shows the results.

**Table 1**

| | | Example 1 | Example 2 | Comparative Example 1 | Comparative Example 2 |
|---|---|---|---|---|---|
| Formulation | BPA328 | 60 part | | 100 part | 60 part |
| | BPF307 | | 60 part | | |
| | Bisphenol A type epoxy resin | 10 part | 10 part | | 10 part |
| | TB2131N | 25 part | 25 part | 30 part | |
| | 290F | | | | 40 part |
| | Epoxy silane coupling agent | 0.14 part | 0.14 part | 0.14 part | 0.14 part |
| Test items | SUS + SUS initial adhesive strength | 50 MPa | 45 MPa | 45 MPa | 35 MPa |
| | SUS + engineering plastic initial adhesive strength | 20 MPa | 20 MPa | 20 MPa | 20 MPa |
| | SUS + SUS adhesive strength after peracetic acid test | 50 MPa | 45 MPa | 23 MPa | 30 MPa |
| | SUS + engineering plastic adhesive strength after peracetic acid test | 20 MPa | 20 MPa | 5 MPa | 10 MPa |
| | SUS + SUS adhesive strength after dipped in hydrogen peroxide solution | 30 MPa | 30 MPa | 10 MPa | Peeled |
| | SUS + engineering plastic adhesive strength after dipped in hydrogen peroxide solution | 10 MPa | 9 MPa | 2 MPa | Peeled |
| | SUS + SUS adhesive strength after autoclaving | 25 MPa | 24 MPa | 15 MPa | Peeled |
| | SUS + engineering plastic adhesive strength after autoclaving | 10 MPa | 11 MPa | 9 MPa | Peeled |

As is apparent from these test results, Examples 1 and 2 well improved in the peracetic acid environment test, hydrogen peroxide solution environment test, and autoclave treatment, compared to Comparative Examples 1 and 2 using no polyamidoamine-based curing agent.

As described above, the present invention prevents deterioration of endoscope parts caused by an acidic liquid chemical and autoclave, and increases the adhesive strength of an endoscope apparatus.

The present invention is not limited to the above examples and can be variously modified without departing from the spirit and scope of the invention when practiced.

## Claims

1. An endoscope apparatus in which parts constituting an endoscope are bonded by an adhesive, **characterized in that** the adhesive is a two-part reaction type adhesive obtained by mixing a base compound in which a fine acrylic rubber powder having an average particle size of not more than 300 nm is dispersed in at least one bisphenol-based epoxy resin selected from the group consisting of a bisphenol A type epoxy resin and a bisphenol F type epoxy resin such that an amount of the fine acrylic rubber powder is 5 to 15 wt% with respect to a weight of the epoxy resin, and a curing agent containing dimer acid, diethylenetriamine, and a diethylenetriamine monomer as main components.

2. An endoscope apparatus in which an end portion of a flexible outer tube of an insertion portion of an endoscope is fixed to an internal member by externally tying the end portion by a thread, and an outer surface of the thread is finished and the thread is fixed by coating the thread with an adhesive in order to ensure insertion properties and prevent a fray of the thread, **characterized in that** the adhesive is a two-part reaction type adhesive obtained by mixing a base compound in which a fine acrylic rubber powder having an average particle size of not more than 300 nm is dispersed in at least one bisphenol-based epoxy resin selected from the group consisting of a bisphenol A type epoxy resin and a bisphenol F type epoxy resin such that an amount of the fine acrylic rubber powder is 5 to 15 wt% with respect to a weight of the epoxy resin, and a curing agent containing dimer acid, diethylenetriamine, and a diethylenetriamine monomer as main components.

3. An endoscope apparatus in which an adhesive is used to raise an adhesive layer around one of an observation lens and an illumination lens of an endoscope, thereby smoothing a corner portion of an edge of the lens, **characterized in that** the adhesive is a two-part reaction type adhesive obtained by mixing a base compound in which a fine acrylic rubber powder having an average particle size of not more than 300 nm is dispersed in at least one bisphenol-based epoxy resin selected from the group consisting of a bisphenol A type epoxy resin and a bisphenol F type epoxy resin such that an amount of the fine acrylic rubber powder is 5 to 15 wt% with respect to a weight of the epoxy resin, and a curing agent containing dimer acid, diethylenetriamine, and a diethylenetriamine monomer as main components.

4. An endoscope apparatus according to any one of claims 1 to 3, **characterized in that** a blending amount of the curing agent with respect to 100 parts by weight of the bisphenol-based epoxy resin is 25 to 30 parts by weight.
